(19) **Europäisches Patentamt / European Patent Office / Office européen des brevets**

(11) **EP 4 745 158 A1**

(12) **EUROPEAN PATENT APPLICATION**

(43) Date of publication:
**20.05.2026 Bulletin 2026/21**

(21) Application number: **25209266.3**

(22) Date of filing: **16.10.2025**

(51) International Patent Classification (IPC):
*C07K 16/28* (2006.01)  *A61K 39/395* (2006.01)

(52) Cooperative Patent Classification (CPC):
**C07K 16/2818;** A61K 39/39591; C07K 2317/24;
C07K 2317/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.08.2025 CN 202511191369**

(71) Applicant: **QILU PHARMACEUTICAL CO., LTD.**
**Jinan, Shandong 250100 (CN)**

(72) Inventors:
• **AN, Zhenming**
**Jinan, Shandong, 250100 (CN)**
• **ZAN, Wenying**
**Jinan, Shandong,, 250100 (CN)**
• **XUE, Jiugang**
**Jinan, Shandong,, 250100 (CN)**

• **YIN, Zhenhao**
**Jinan, Shandong, 250100 (CN)**
• **FAN, Jiaxin**
**Jinan, Shandong, 250100 (CN)**
• **SUN, Lixia**
**Jinan, Shandong, 250100 (CN)**
• **LI, Daoyuan**
**Jinan, Shandong, 250100 (CN)**
• **LIU, Chuanlei**
**Jinan, Shandong, 250100 (CN)**
• **ZHANG, Feng**
**Jinan, Shandong, 250100 (CN)**

(74) Representative: **Bandpay & Greuter**
**11, rue Christophe Colomb**
**75008 Paris (FR)**

Remarks:
The complete document including Reference Table(s) and the Sequence Listing(s) can be downloaded from the EPO website

(54) **A HIGH-CONCENTRATION COMPOSITION OF X AND GLUCURONIDATION**

(57) Provided are a high-concentration composition of PD-1 antibody and a preparation method thereof. The composition comprises Pembrolizumab, where a portion of Pembrolizumab comprises a glucuronidation modification at the lysine at position 27 of a light chain having the amino acid sequence set forth in SEQ ID NO:2, and where the content of the light chain comprising the glucuronidation modification of the Pembrolizumab in the composition is less than or equal to about 3%, expressed as a percentage of the content of the light chain of Pembrolizumab comprising the glucuronidation modification accounting for the total content of light chains of Pembrolizumab in the composition. Also provided is a pharmaceutical formulation comprising the composition. Additionally, also provided are a method for detecting Pembrolizumab comprising the glucuronidation modification as well as its use in quality inspection or quality control of a Pembrolizumab-containing product.

**EP 4 745 158 A1**

## Description

### Cross-Reference to Related Application

This application claims priority to Chinese Patent Application No.: CN 202511191369.X, filed on August 25, 2025, the entire contents of which are incorporated herein by reference for all purposes.

### Technical Field

The present application relates to the field of pharmaceutical formulations. In particular, the present application provides a high-concentration composition of PD-1 antibody and a preparation method thereof.

### Background of the Invention

With the continued development of therapeutic medicines of recombinant proteins, there is an increasingly urgent need to achieve subcutaneous administration of high-concentration medicines. In particular, in the field of treatment of chronic diseases such as tumors, there is an urgent need to develop antibody formulations with high concentration and small volume, in order to improve convenience of medication for patients and compliance of outpatients to the medication. Therefore, high-concentration dosage forms for subcutaneous injection have become one of the key development directions of differential research and development for therapeutic medicines of recombinant proteins.

Under normal physiological conditions, programmed death receptor 1 (PD-1) and programmed death ligand 1 (PD-L1) are important negative immunoregulatory factors that inhibit T cell activation and mediate the occurrence of infectious diseases and autoimmune diseases, and immune escape of tumor cells. However, during tumorigenesis, tumor cells abnormally express PD-L1, which transmits an inhibitory signal by binding to PD-1 on surface of T cells, thereby inhibiting activation and proliferation of the T cells, thus enabling the tumor cells to escape the surveillance and attack of the immune system and achieve the immune escape.

Pembrolizumab is a humanized monoclonal antibody that specifically binds to the PD-1 receptor on the surface of T cells and blocks the interaction between PD-1 and its ligands such as PD-L1, thereby relieving the immunosuppression of the tumor cells on the T cells, restoring the anti-tumor activity of the T cells, enhancing anti-tumor immune responses of the body, and achieving tumor cell killing.

Although high-concentration formulations have been developed for some antibody drugs, the production of high-concentration antibody formulations (e.g., at ≥100 mg/mL) is still confronted with significant challenges, including irreversible aggregation phenomena, irreversible precipitation and/or high viscosity issues, as well as the generation of potential new impurities. As a medication, Pembrolizumab must maintain its stability and efficacy. Quality control of pharmaceutical compositions mainly focuses on the control of content of active ingredient as well as contents of related substances (such as variants or impurities). In particular, the contents of related substances need to meet stringent pharmaceutical standards.

### Summary of the Invention

In a first aspect, the present application provides a composition comprising Pembrolizumab, wherein a portion of Pembrolizumab comprises a glucuronidation modification at the lysine at position 27 of a light chain having the amino acid sequence set forth in SEQ ID NO:2, and wherein the content of the light chain comprising the glucuronidation modification of Pembrolizumab in the composition is less than or equal to about 3%, expressed as a percentage of the content of the light chain comprising the glucuronidation modification of Pembrolizumab accounting for the total content of light chains of Pembrolizumab in the composition (e.g., which is calculated based on trypsin-cleaved peptide mass fingerprinting analysis).

In a second aspect, the present application provides a pharmaceutical formulation comprising the composition of the first aspect, and one or more pharmaceutically acceptable carriers.

In a third aspect, the present application provides a method of detecting Pembrolizumab comprising a glucuronidation modification in a Pembrolizumab-containing composition or pharmaceutical formulation, wherein the glucuronidation modification is located at the lysine at position 27 of a light chain having the amino acid sequence set forth in SEQ ID NO:2 of Pembrolizumab, the method comprising:

subjecting the composition or pharmaceutical formulation to enzymatic cleavage with trypsin to obtain an enzymatically cleaved product; and
subjecting the enzymatically cleaved product to a peptide mass fingerprinting analysis, e.g., a peptide mass fingerprinting analysis using LC-MS/MS, and determining the presence or absence of Pembrolizumab comprising the glucuronidation modification based on the first-order and second-order mass spectra.

In a fourth aspect, the present application provides a method for quality inspection or quality control of a Pembrolizumab-containing product, comprising detecting the content of Pembrolizumab comprising a glucuronidation modification in the Pembrolizumab-containing product, wherein the glucuronidation modification is located at the lysine at position 27 of a light chain having the amino acid sequence set forth in SEQ ID NO:2 of Pembrolizumab.

In a fifth aspect, the present application provides use of Pembrolizumab comprising a glucuronidation modification for quality inspection or quality control of a Pembrolizumab-containing product, wherein the glucuronidation modification is located at the lysine at position 27 of a light chain having the amino acid sequence set forth in SEQ ID NO:2 of Pembrolizumab.

In some embodiments, the composition, the pharmaceutical formulation, or the Pembrolizumab-containing product comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab.

**Brief Description of the Drawings**

FIG. 1 shows an extracted ion chromatogram (XIC) of a peptide segment comprising a glucuronidation modification of Pembrolizumab, ASK$^{27}$GVSTSGYSYLHWYQQKPGQAPR (SEQ ID NO:3).

FIG. 2 shows a first-order mass spectrum of the peptide segment comprising the glucuronidation modification of Pembrolizumab, ASK$^{27}$GVSTSGYSYLHWYQQKPGQAPR (SEQ ID NO:3).

FIG. 3 shows a second-order mass spectrum of the peptide segment comprising the glucuronidation modification of Pembrolizumab, ASK$^{27}$GVSTSGYSYLHWYQQKPGQAPR (SEQ ID NO:3). The peptide segment comprising the glucuronidation modification is susceptible to neutral loss upon HCD fragmentation. "*" in FIG. 3 indicates a fragment ion of +121.99Da resulting from the neutral loss after the glucuronidation modification occurs at the lysine of the peptide segment.

**Description of Sequences**

SEQ ID NO:1 is the amino acid sequence of two identical heavy chains of Pembrolizumab, and is shown as below (note that the underlined portions are CDR domains annotated according to the Kabat numbering scheme):

QVQLVQSGVEVKKPGASVKVSCKASGYTFT<u>NYYMY</u>WVRQAPGQGLEW

MG<u>GINPSNGGTNFNEKFKN</u>RVTLTTDSSTTTAYMELKSLQFDDTAVYYCAR<u>R</u>

<u>DYRFDMGFDY</u>WGQGTTVTVSSASTKGPSVFPLAPCSRSTSESTAALGCLVKDY

FPEPVTVSWNSGALTSGVHTFPAVLQSSGLYSLSSVVTVPSSSLGTKTYTCNVD

HKPSNTKVDKRVESKYGPPCPPCPAPEFLGGPSVFLFPPKPKDTLMISRTPEVTC

VVVDVSQEDPEVQFNWYVDGVEVHNAKTKPREEQFNSTYRVVSVLTVLHQD

WLNGKEYKCKVSNKGLPSSIEKTISKAKGQPREPQVYTLPPSQEEMTKNQVSL

TCLVKGFYPSDIAVEWESNGQPENNYKTTPPVLDSDGSFFLYSRLTVDKSRWQ

EGNVFSCSVMHEALHNHYTQKSLSLSLGK

SEQ ID NO:2 is the amino acid sequence of two identical light chains of Pembrolizumab, and is shown as below (note that the underlined portions are CDR domains annotated according to the Kabat numbering scheme):

EIVLTQSPATLSLSPGERATLSCRASKGVSTSGYSYLHWYQQKPGQAPRLL

IYLASYLESGVPARFSGSGSGTDFTLTISSLEPEDFAVYYCQHSRDLPLTFGGGT

KVEIKRTVAAPSVFIFPPSDEQLKSGTASVVCLLNNFYPREAKVQWKVDNALQ

SGNSQESVTEQDSKDSTYSLSSTLTLSKADYEKHKVYACEVTHQGLSSPVTKSF

NRGEC

**Detailed Description**

In the specification and the claims, the term "Pembrolizumab" refers to an antibody formed from two identical heavy chains and two identical light chains joined by disulfide bonds, wherein the sequence of the two identical heavy chains is set forth in SEQ ID NO:1, while the sequence of the two identical light chains is set forth in SEQ ID NO:2.

In the specification and the claims, the term "about" refers to a range of values considered by one of ordinary skill in the art to be equivalent to the recited value (e.g., having the same function or result), e.g., +/-10% of the recited value.

In the specification and the claims, the terms "including", "comprising" and "containing" mean "comprising but not limited to", and do not intend to exclude other parts, additives, components or steps.

The inventors of the present application have surprisingly found that Pembrolizumab comprising a modification, e.g., Pembrolizumab comprising a glucuronidation modification, e.g., a glucuronidation modification located at the lysine at position 27 of a light chain having the amino acid sequence of Pembrolizumab ($K^{27}$, located in the CDR1 region of the light chain), was produced during the preparation of Pembrolizumab.

In some embodiments, the content of the light chain comprising the glucuronidation modification of Pembrolizumab is less than or equal to about 3% of the total content of light chains of Pembrolizumab in the composition, without affecting the binding activity and biological activity of the Pembrolizumab sample. Thus, in these embodiments, if the content of the light chain comprising of the glucuronidation modification of Pembrolizumab is evaluated to be no more than about 3% during the preparation of Pembrolizumab, there is no need to perform operations of removing variants or impurities in general, which simplifies the production process and saves production costs to some extent.

In a first aspect, the present application provides a composition comprising Pembrolizumab, wherein a portion of Pembrolizumab comprises a glucuronidation modification at the lysine at position 27 of a light chain having the amino acid sequence set forth in SEQ ID NO:2.

In some embodiments of the first aspect, the composition comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab.

In some embodiments of the first aspect, the content of the light chain comprising the glucuronidation modification of Pembrolizumab in the composition is less than or equal to about 3%, expressed as a percentage of the content of the light chain comprising the glucuronidation modification of Pembrolizumab accounting for the total content of light chains of Pembrolizumab in the composition, which is calculated based on trypsin-cleaved peptide mass fingerprinting analysis.

In some embodiments of the first aspect, the content of the light chain comprising the glucuronidation modification of Pembrolizumab in the composition is greater than or equal to about 0.001%, greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2.0%, or greater than or equal to about 2.5%, expressed as a percentage of the content of the light chain comprising the glucuronidation modification of Pembrolizumab accounting for the total content of light chains of Pembrolizumab in the composition.

In some embodiments of the first aspect, the content of the light chain comprising the glucuronidation modification of Pembrolizumab in the composition is less than or equal to about 3.0%, less than or equal to about 2.9%, less than or equal to about 2.8%, less than or equal to about 2.7%, less than or equal to about 2.6%, less than or equal to about 2.5%, less than or equal to about 2.4%, less than or equal to about 2.3%, less than or equal to about 2.2%, less than or equal to about 2.1%, less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, less than or equal to about 0.1%, less than or equal to about 0.05%, or less than or equal to about 0.01%, expressed as a percentage of the content of the light chain comprising the glucuronidation modification of Pembrolizumab accounting for the total content of light chains of Pembrolizumab in the composition.

In some embodiments of the first aspect, the content of the light chain comprising the glucuronidation modification of Pembrolizumab in the composition is from 0.001% to 3.0%, from 0.01% to 3.0%, from 0.05% to 3.0%, from 0.1% to 3.0%, from 0.2% to 3.0%, from 0.3% to 3.0%, from 0.4% to 3.0%, from 0.5% to 3.0%, from 0.6% to 3.0%, from 0.7% to 3.0%, from 0.8% to 3.0%, from 0.9% to 3.0%, from 1.0% to 3.0%, from 1.1% to 3.0%, from 1.2% to 3.0%, from 1.3% to 3.0%, from 1.4% to 3.0%, from 1.5% to 3.0%, from 1.6% to 3.0%, from 1.7% to 3.0%, from 1.8% to 3.0%, from 1.9% to 3.0%, from 2.0% to 3.0%, from 2.1% to 3.0%, from 2.2% to 3.0%, from 2.3% to 3.0%, from 2.4% to 3.0%, from 2.5% to 3.0%, from 2.6% to 3.0%, from 2.7% to 3.0%, from 2.8% to 3.0%, from 2.9% to 3.0%, or any interval within any of the foregoing ranges or any value within said interval, expressed as a percentage of the content of the light chain comprising the glucuronidation modification of Pembrolizumab accounting for the total content of light chains of Pembrolizumab in the composition.

In some embodiments of the first aspect, the content of the light chain comprising the glucuronidation modification of Pembrolizumab in the composition is from 0.08% to 2.59%, e.g., 0.08%, 0.67%, 1.30%, 2.59%, or any of preceding values or any interval within any of the foregoing ranges or any value within said interval, expressed as a percentage of the content of the light chain comprising the glucuronidation modification of Pembrolizumab accounting for the total content of light chains of Pembrolizumab in the composition.

It is to be understood that although the above reference to the content of Pembrolizumab comprising the glucuronidation modification is expressed as a percentage of the content of the light chain comprising the glucuronidation modification accounting for the total content of all light chains in the composition, this is only one way of expression. It will be appreciated by those skilled in the art that the content of Pembrolizumab comprising the glucuronidation modification can also be expressed in a variety of other ways, such as by the percentage of the content of a peptide segment comprising the glucuronidation modification (which may be referred to as a "modified peptide segment") in the composition accounting for the sum of the content of the peptide segment comprising the glucuronidation modification and the content of corresponding unmodified peptide segment (e.g., having the same or substantially the same amino acid sequence but excluding the modification) in the composition.

Those skilled in the art can measure and determine the content of Pembrolizumab comprising the glucuronidation modification in the composition in a variety of ways. For example, in some embodiments, the content can be measured and determined by the following method:

(a) adding urea at a final concentration of 6 M and 10 mM of TCEP to a certain amount of sample, performing incubation at 37°C for 20 min for denaturation and reduction, followed by addition of a final concentration of 20 mM of IAM, and incubation at 37°C for 15 min for alkylation;

(b) then adding a solution of 50 mM ammonium bicarbonate to dilute urea to a concentration of 1 M, followed by adding sequencing-grade trypsin at a ratio of protein: enzyme = 10:1 (mass ratio) to perform enzymatic cleavage at 37°C for 25 min, and at the end of the enzymatic cleavage, adding formic acid at a final concentration of 1% to stop the enzymatic cleavage; and

(c) subjecting the sample to centrifugation and then peptide mass fingerprint analysis with liquid chromatography-tandem high-resolution mass spectrometry, in which peptide segments are separated with a C18 column and then analyzed with a first-order mass spectrometry and a second-order mass spectrometry. Then, XICs of the peptide segment comprising the glucuronidation modification (ASK[27]GVSTSGYSYLHWYQQKPGQAPR (SEQ ID NO:3), with mass-to-charge ratios of main ions of 743.86 and 595.29, respectively) and the corresponding unmodified peptide segment (GVSTSGYSYLHWYQQKPGQAPR (SEQ ID NO:4), with the mass-to-charge ratios of main ions of 628.31 and 837.41, respectively) are extracted respectively, and the mass deviation is set to 20 ppm. Peak areas of the respective target components are obtained by integration, and the modification ratio is calculated according to the following formula:

$$\text{modification ratio} = \frac{\text{XIC peak area of modified peptide segment}}{\text{XIC peak area of moified peptide segment} + \text{XIC area of unmodified peptide segment}} \times 100\%$$

In a second aspect, the present application provides a pharmaceutical formulation comprising the composition of the first aspect, and one or more pharmaceutically acceptable carriers.

The pharmaceutically acceptable carrier is non-toxic to a recipient at the dosages and concentrations employed. In some embodiments, the pharmaceutically acceptable carrier includes, for example, water; a buffer, such as phosphate, citrate and another organic acid; an antioxidant, such as ascorbic acid and methionine; a preservative; a hydrophilic polymer, such as polyvinylpyrrolidone; a chelating agent, such as EDTA, and the like.

In some embodiments, the pharmaceutical formulation may further comprise one or more of the following: a lubricant, such as talc, magnesium stearate, and mineral oil; a wetting agent; an emulsifier; a suspending agent; a preservative, such as benzoic acid, sorbic acid and calcium propionate; a sweetening agent and/or a flavoring agent, and the like.

In some embodiments, the composition, the pharmaceutical formulation, and/or the Pembrolizumab-containing product in the present application may comprise one or more of histidine, sucrose, L-arginine, methionine, polysorbate 80, and hyaluronidase.

In some embodiments, the composition, the pharmaceutical formulation, and/or the Pembrolizumab-containing product in the present application may be formulated in a form of a tablet, a pill, a powder, a lozenge, an elixir, a suspension, an emulsion, a solution, a syrup, a suppository, a capsule, or the like.

In some embodiments, the composition, the pharmaceutical formulation, and/or the Pembrolizumab-containing product in the present application may be delivered with any physiologically acceptable administration mode which includes, but is not limited to, oral administration, parenteral administration, nasal administration, rectal administration, intraperitoneal administration, intravascular injection, subcutaneous administration, transdermal administration, inhalation administration, and the like.

In some embodiments, the pharmaceutical formulation for *in vivo* administration must be sterilized. This can be easily achieved by using a sterile filter membrane for filtration.

In some embodiments, the pharmaceutical formulation for therapeutic use may be formulated in the form of a lyophilized formulation or aqueous solution for storage by mixing a reagent of the desired purity with a pharmaceutically acceptable carrier, an excipient, and the like, as appropriate.

In a third aspect, the present application provides a method of detecting Pembrolizumab comprising a glucuronidation modification in a Pembrolizumab-containing composition or pharmaceutical formulation, wherein the glucuronidation modification is located at the lysine at position 27 of a light chain in the amino acid sequence set forth in SEQ ID NO:2 of Pembrolizumab, the method comprising:

subjecting the composition or pharmaceutical formulation to enzymatic cleavage with trypsin to obtain an enzymatically cleaved product; and
subjecting the enzymatically cleaved product to a peptide mass fingerprinting analysis, e.g., a peptide mass fingerprinting analysis using LC-MS/MS, and determining the presence or absence of Pembrolizumab comprising the glucuronidation modification based on the first-order and second-order mass spectra.

In some embodiments of the third aspect, the content of Pembrolizumab or the content of Pembrolizumab comprising the glucuronidation modification in the Pembrolizumab-containing composition or pharmaceutical formulation is as described in the first aspect.

In some embodiments of the third aspect, the method further comprises obtaining extracted ion chromatograms (XICs) of a peptide segment comprising the glucuronidation modification and an unmodified peptide segment after the peptide mass fingerprint analysis, and calculating the content of Pembrolizumab comprising the glucuronidation modification according to corresponding peak areas.

In a fourth aspect, the present application provides a method for quality inspection or quality control of a Pembrolizumab-containing product, wherein a portion of Pembrolizumab comprises a glucuronidation modification at the lysine at position 27 of a light chain having the amino acid sequence set forth in SEQ ID NO:2, the method comprising detecting the content of Pembrolizumab comprising the glucuronidation modification in the Pembrolizumab-containing product.

In some embodiments, the content of the light chain comprising the glucuronidation modification of Pembrolizumab in the composition is less than or equal to about 3.0%, less than or equal to about 2.9%, less than or equal to about 2.8%, less than or equal to about 2.7%, less than or equal to about 2.6%, less than or equal to about 2.5%, less than or equal to about 2.4%, less than or equal to about 2.3%, less than or equal to about 2.2%, less than or equal to about 2.1%, less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, less than or equal to about 0.1%, less than or equal to about 0.05%, or less than or equal to about 0.01%, expressed as a percentage of the content of the light chain comprising the glucuronidation modification of Pembrolizumab accounting for the total content of light chains of Pembrolizumab in the composition, indicating that the product meets pharmaceutical requirements.

In a fifth aspect, the present application provides the use of Pembrolizumab comprising a glucuronidation modification in quality inspection or quality control of a Pembrolizumab-containing product, wherein the glucuronidation modification is located at the lysine at position 27 in the amino acid sequence set forth in SEQ ID NO:2 of Pembrolizumab.

In some embodiments of the fourth and/or the fifth aspect, the content of Pembrolizumab or Pembrolizumab comprising the glucuronidation modification in the product is as described in the first aspect.

In some embodiments, the present application achieves at least one or more of the following advantageous technical effects.

1. The glucuronidation modification included in a portion of Pembrolizumab in the composition of the present application is located in a CDR in the antibody light chain (at the lysine at position 27 of a light chain of the amino acid sequence set forth in SEQ ID NO:2). Therefore, as compared with an unmodified Pembrolizumab, Pembrolizumab comprising the glucuronidation modification has affected biological activity and function (including, for example, antigen-binding activity). The present application, however, demonstrates that there is no significant change in the overall biological activity and function of the composition when the content of Pembrolizumab comprising the glucuronidation modification in the composition is less than or equal to about 3% of the total content of Pembrolizumab in the composition.

2. The present application demonstrates that if the content of the light chain of Pembrolizumab comprising of the glucuronidation modification is evaluated to be no more than about 3% during the preparation of Pembrolizumab (expressed as a percentage of the content of the light chain comprising the modification accounting for the total content of the light chain comprising the modification and the unmodified light chain), there is no need to perform operations of removing variants or impurities in general, which simplifies the production process and saves production costs to some extent.

It is to be understood that the features, characteristics, components, or steps described in a particular aspect, embodiment, or example of the present application may be applied to any other aspect, embodiment, or example described herein unless in conflict with them.

The above disclosure generally describes the present invention, which is further exemplified by the following examples. These examples are described merely to illustrate the invention and are not intended to limit the scope of the invention. Although specific terms and values are used herein, such terms and values are also to be understood as exemplary and do not limit the scope of the invention. Unless otherwise specified, the experimental methods and techniques in this specification are those well known to those skilled in the art.

**Examples**

**Example 1 Preparation of Pembrolizumab**

**Upstream cell culture process**

In this example, an expression system, the CHOZN®GS expression system from Merck, was employed, which included a CHO-K1 host cell for cGMP library construction. The expression vector was pCGS3.2 plasmid from Merck that enabled efficient expression of exogenous recombinant proteins in mammal cells. The medium system included EX-CELL® CD CHO Fusion medium, QL001 medium, cell boost5 medium, and XF04 High-Performance feed medium from OPM Biosciences.

In accordance with typical cell culture processes for antibody drugs, the production of a stock solution of Pembrolizumab was carried out according to a process of cell recovery, step-by-step expansion in a shake flask and a bioreactor, and production in a 500 L disposable bioreactor, in which a feeding batch culture process was used in the production stage.

Cells in a cell cryopreservation tube taken from a working cell bank were thawed in a water bath at 37°C, cultured in a shake-flask expansion medium, successively expanded in a shake flask and a bioreactor, and inoculated into a 500 L disposable bioreactor at a target density of $(4.5\pm1.0) \times 10^6$ cells/ml.

The temperature was set to 36.5°C in the growth stage, and cooled down to 30°C in the expression stage. The pH was set to $7.0\pm0.3$, the dissolved oxygen was set to 40% and the stirring speed was 50-70 rpm. Air was supplied from the top at a rate of (0-15) L/min, and from the bottom at a rate of (0-10) L/min. pH control was coupled with bottom aeration (carbon dioxide) and supply of sodium carbonate solution, and dissolved oxygen control was coupled with bottom aeration (oxygen). During the culture process, the feed medium was added in a volume which was 28-48% of the initial volume. A glucose solution was added as needed for cell growth, and an antifoaming agent solution was supplemented as needed for culture. The culture period was 16 days. The cell culture was subjected to deep filtration and sterilization by 0.2 $\mu$m filtration, and the supernatant was collected for purification.

**Downstream purification process**

In this example, typical purification processes for antibody drugs were employed, including affinity chromatography, low pH viral inactivation, anion exchange chromatography, cation exchange chromatography, virus removal filtration, ultrafiltration concentration, and finally addition of excipients and sterilization and filtration so as to obtain a stock solution.

Protein A affinity chromatography. Protein A affinity chromatography was used as the primary capture step, using AT Protein A Diamond plus fillers from the manufacturer Bestchrom. The target antibody was bound to the fillers, while components of the culture medium and host cell proteins as impurities were not bound. After equilibrating the column with a Tris solution, the loading phase was started. At the end of the loading, the column was rinsed, to collect a solution of the eluted protein.

Low pH viral inactivation. The eluate solution from the protein A affinity chromatography was adjusted to a low pH with acetic acid, to inactivate viruses that may be present. The product after the viral inactivation was neutralized with a Tris solution and filtered through a deep filter and a sterilizing filter, to obtain a sample from the viral inactivation and neutralization filtration, which was further processed in a subsequent step of anion exchange chromatography.

Anion exchange chromatography. Toyopearl® NH2-750F fillers from TOSOH were used to perform the anion chromatography. They were equilibrated with a Tris buffer, and loaded with the product from the viral inactivation and neutralization filtration. The target protein flowed through the chromatography column during the loading. During the loading and the equilibrium process followed the loading, the target protein was collected according to a collection interval, to obtain a solution collected from the anion exchange chromatography.

Cation exchange chromatography. The cation chromatography was carried out with Capto SP ImpRes fillers from Cytiva, and the cation exchange chromatography column was equilibrated with a sodium acetate-acetic acid buffer. The protein solution collected from the anion exchange chromatography was loaded, allowing the protein to bind onto the cation exchange chromatography fillers. At the end of the loading, the target protein was eluted from the cation exchange chromatography column by the way of gradient elution, to collect a solution of the eluted protein.

Virus removal filtration. The solution collected from the cation exchange chromatography was subjected to virus removal filtration with a virus removal prefilter (A1HC, as a cellulose-diatomaceous earth material) and a virus removal filter (Viresolve® Pro, as a PES material). At the end of filtration, pipelines and filters were overwashed with a buffer, and the filtered sample was collected for sterilization filtration.

Ultrafiltration concentration. The antibody was adjusted to a desired concentration by ultrafiltration concentration. Upon completion of the ultrafiltration concentration, the excipient to be added was added to the antibody solution. Finally the concentration of the protein was adjusted to about 165 mg/ml. The final product additionally contained 10 mM histidine, 2% sucrose, 2% L-arginine, 15 mM methionine, 0.4 mg/ml polysorbate 80, pH 5.5, and 2000 IU/ml hyaluronidase. The sample was sterilized and filtered through a 0.22 $\mu$m filter, filled into a 2-ml sterile molded vial under a controlled condition, and sealed with a film-coated rubber stoppers and aluminum-plastic composite cap.

**Example 2 Discovery of glucuronidation modification in Pembrolizumab**

The sample prepared in Example 1 was subjected to liquid chromatography-tandem high-resolution mass spectrometry with the following steps.

The sample was added with urea at a final concentration of 6 M and 10 mM TCEP and incubated at 37°C for 20 min for denaturation and reduction. At the end of the incubation, iodoacetamide (IAM) was added at a final concentration of 20 mM. Incubation was performed at 37°C for 15 min in the dark for alkylation. The urea was then diluted to a concentration of 1 M by the addition of a solution of 50 mM ammonium bicarbonate. Sequencing-grade trypsin (Manufacturer: Rhinogen, Catalog No.: QIP-003A) was added at a ratio of protein: enzyme = 10:1 (mass ratio) and enzymatic cleavage was performed at 37°C for 25 min. At the end of the enzymatic cleavage, formic acid was added at the final concentration of 1% to stop the enzymatic cleavage. Centrifugation was performed at 12000 rpm for 3 min and the supernatant was transferred to a sample vial.

The sample to be tested was analyzed with liquid chromatography-tandem high-resolution mass spectrometry with an injection volume of 10 $\mu$g. Targeted collection of information from first-order and second-order mass spectrometry of the peptide segment comprising the glucuronidation modification (peptide sequence: ASK[27]GVSTSGYSYLH-WYQQKPGQAPR (SEQ ID NO:3) in CDR1 of the light chain in its main ionic form (m/z ratio of 743.86) was performed. Conditions for chromatography and mass spectrometry employed were as follows.

Conditions for chromatography

| LC system | UHPLC, Vanquish horizon, Thermo |
|---|---|
| Chromatography column | ACQUITY Premier BEH C18 column, 1.7 $\mu$m particle size, 2.1$\times$100 mm |
| Colum temperature | 60°C |
| Sample chamber temperature | 8°C |
| Mobile phase | A: a solution of 0.1% formic acid/water; B: a solution of 0.08% formic acid/80% acetonitrile/20% water |
| Elution gradient | After 1% phase B was used for equilibration for 3 min, phase B increased linearly to 45% in 57 min |

Conditions for mass spectrometry

| Mass spectrometer | Orbitrap Exploris 480, Thermo |
|---|---|
| Ionization mode | H-ESI positive |
| Capillary voltage | 3.8 kV |
| Ion transfer tube temperature | 320°C |
| Vaporizer temperature | 300°C |
| Resolution of first-order mass spectrometry | 90000 |
| AGC Target of first-order mass spectrometry | 1e5 |
| Resolution of second-order mass spectrometry | 15000 |
| ACG Target of first-order mass spectrometry | 1e5 |
| HCD Collision Energy (NCE) | 27% |

Data analysis was performed with Xcalibur software.

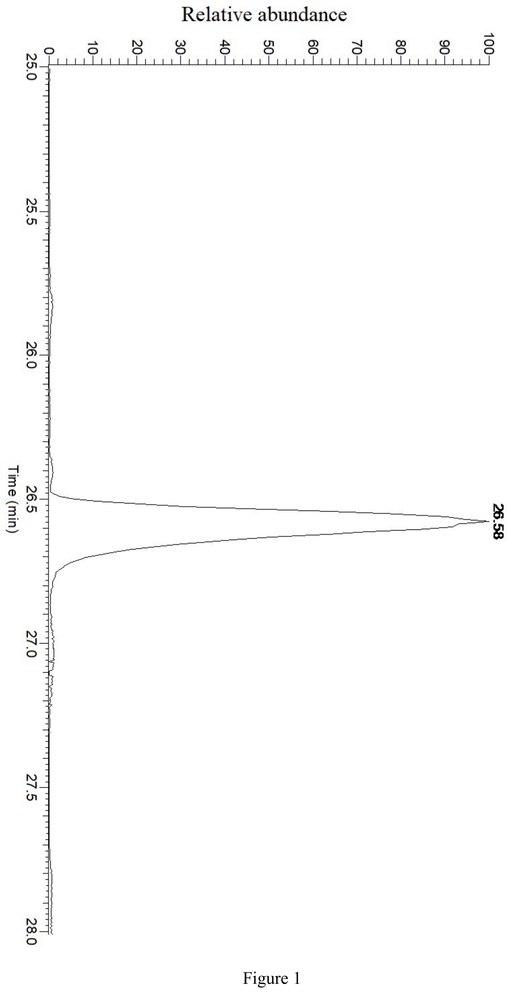

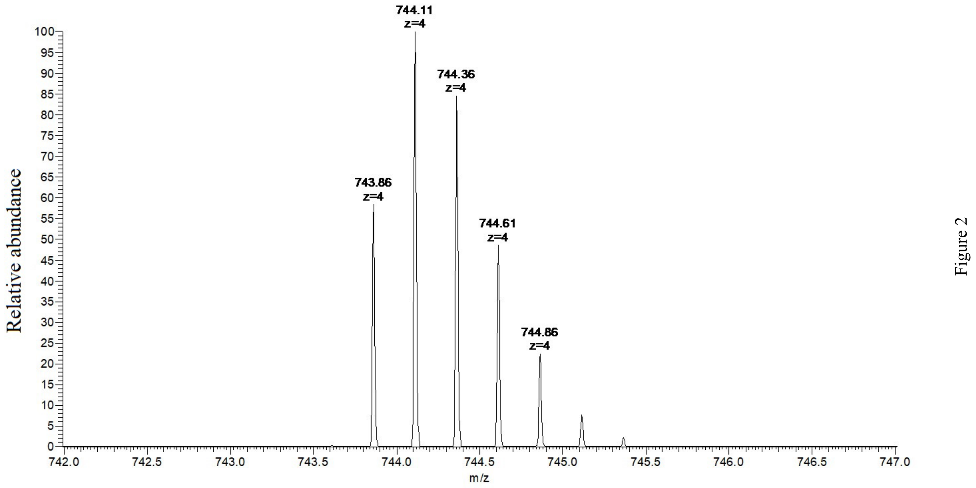

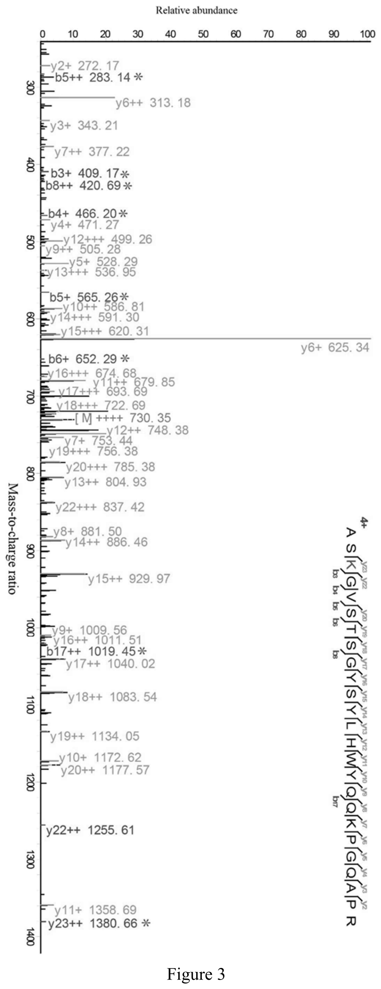

Figures 1 to 3 show the extracted ion chromatogram (XIC) (FIG. 1), the first-order mass spectrum (FIG. 2) and the second-order mass spectrum (FIG. 3), respectively, of the modified peptide segment.

The results of the above analysis indicate that the glucuronidation modification was present in the lysine at position 27 of the light chain (LC:K[27]) of Pembrolizumab.

**Example 3. Preparation of forced glucuronidation samples of Pembrolizumab and determination of content thereof**

In this example, an artificial glucuronidation was performed on Pembrolizumab using glucuronic acid to obtain glucuronidation samples of Pembrolizumab (referred to herein as "forced glucuronidation samples" or "forced samples").

A commercial glucuronic acid reagent was used, and Pembrolizumab was placed in 128 mM of a glucuronic acid solution, and incubated at 37°C for 6 h, 12 h and 24 h, respectively. At the end of the incubation, the forced incubation samples were subjected to medium exchange with formulation excipients.

The forced samples were then determined for ratios of the glucuronidation modification at the LC:K[27] site with peptide mass fingerprint analysis. Steps of the analysis were as follows.

About 150 $\mu$g of each sample was added with urea at a final concentration of 6 M and 10 mM TCEP and incubated at 37°C for 20 min for denaturation and reduction. An IAM at a final concentration of 20 mM was added, followed by incubation at 37°C for 15 min in the dark for alkylation. Urea was then diluted to a concentration of 1 M by the addition of the solution of 50 mM ammonium bicarbonate. The sequencing-grade trypsin (Manufacturer: Rhinogen, Catalog No.: QIP-003A) was added at a ratio of protein: enzyme = 10: 1 (mass ratio) and enzymatic cleavage was performed at 37°C for 25 min. At the end of the enzymatic cleavage, formic acid was added at the final concentration of 1% to stop the enzymatic cleavage. Centrifugation was performed at 12000 rpm for 3 min and the supernatant was transferred to a sample vial.

The sample to be tested was analyzed with liquid chromatography-tandem high-resolution mass spectrometry with an injection volume of 3 $\mu$g. Conditions for chromatography and mass spectrometry employed were as follows.

Conditions for chromatography:

| LC system | UHPLC, Vanquish Flex, Thermo |
|---|---|
| Chromatography column | ACQUITY Premier BEH C18 column, 1.7 $\mu$m particle size, 2.1×100 mm |
| Colum temperature | 60°C |
| Sample chamber temperature | 8°C |
| Mobile phase | A: a solution of 0.1% formic acid/water; B: a solution of 0.08% formic acid/80% acetonitrile/20% water |
| Elution gradient | After 1% phase B was used for equilibration for 3 min, phase B increased linearly to 45% in 57 min |

Conditions for mass spectrometry:

| MS system | Q-Exactive Plus, Thermo |
|---|---|
| Ionization mode | ESI positive |
| Scan range of first-order mass spectrometry | 200-2000m/z |
| Capillary voltage | 3.2kV |
| Capillary Temp | 320°C |
| Resolution of first-order mass spectrometry | 70000 |
| AGC Target of first-order mass spectrometry | $1e^6$ |
| Resolution of second-order mass spectrometry | 17500 |
| AGC Target of second-order mass spectrometry | $1e^5$ |
| Selected ion window of second-order mass spectrometry | 1.6 m/z |

Data analysis was performed by Byos software from Protein Metrics. XICs of two mostly responsive charge forms of the modified peptide segment (ASK[27]GVSTSGYSYLHWYQQKPGQAPR (SEQ ID NO:3), with mass-to-charge ratios of main ions of 743.86 and 595.29, respectively) and its corresponding unmodified peptide segment (GVSTSGYSYLHWYQQKPGQAPR (SEQ ID NO:4), with the mass-to-charge ratios of main ions of 628.31 and 837.41, respectively) were extracted. Peak areas of the respective target components were obtained by integration, and the modification ratio was calculated according to the following formula:

$$\text{modification ratio} = \frac{\text{XIC peak area of modified peptide segment}}{\text{XIC peak area of moified peptide segment} + \text{XIC area of unmodified peptide segment}} \times 100\%$$

The modification ratios for the respective samples are shown in Table 1 below. The results of the analysis show that the ratio of the glucuronidation modification at LC:K[27] site reached 2.59% after incubation of Pembrolizumab in the glucuronic acid solution at 37°C for 24 hours.

Table 1. Ratios of components with glucuronidation modification at LC:K[27] in forced samples

| Sample type | Modification ratio (%) |
|---|---|
| Control sample (Pembrolizumab prepared in Example 1) | 0.08 |
| Sample incubated at 37°C for 6 h | 0.67 |
| Sample incubated at 37°C for 12 h | 1.30 |

(continued)

| Sample type | Modification ratio (%) |
|---|---|
| Sample incubated at 37°C for 24 h | 2.59 |

### Example 4. Determination of activities of forced glucuronidation samples of Pembrolizumab

The binding activities and biological activities of the samples prepared in Example 1 and Example 3 were studied in this example.

The abilities of the above test samples to compete with PD-L1 for PD-1 binding were measured by time-resolved fluorescence resonance energy transfer (TR-FRET) as follows.

Firstly, serial dilutions of the control sample and test samples were added to a 96-well shallow plate at 5μl/well, and diluted PD-1 (His-tagged) protein and biotin-labeled PD-L1 protein were added to the plate, each at 5μl/well. Then a 100-fold diluted mixture of streptavidin-d2 and MAb Anti-6HIS-Tb cryptate Gold were mixed in equal volumes and added into the 96-well shallow plate at 10 μl/well. The plate was sealed with a sealing film and incubated at room temperature for 2 hours. The incubated 96-well shallow plate was centrifuged briefly at 1000 rpm, and readings were taken using the time-resolved fluorescence function of a microplate reader (SPARK Multifunctional Microplate Reader from TECAN), at an excitation wavelength of 320nm and emission wavelengths of 620nm, 665nm.

With sample concentrations as the X-axis, and TR-FRET ratios (signal values at 665 nm ÷ signal values at 620 nm × 10000, automatically calculated by software) as the Y-axis, a four-parameter fitting was performed to calculate the results.

With the sample prepared in Example 1 as control (the PD-1 binding activity of the control sample was set as 100%), the binding activities of the forced glucuronidation samples prepared in Example 3 were determined. Results of the activity determination are shown in Table 2. The results of the analysis show that the binding activities of the respective samples to PD-1 were between 80-120% of the control sample.

Table 2. Results of determination of binding activities of forced glucuronidation samples

| Sample type | PD-1 binding activity (%) |
|---|---|
| Control sample | 100 |
| Sample incubated at 37°C for 6 h | 105 |
| Sample incubated at 37°C for 12 h | 109 |
| Sample incubated at 37°C for 24 h | 105 |

The biological activities of the respective samples were determined by a reporter-gene method. CHO cells transfected with PD-L1 and an anti-CD3-single-chain antibody fragment (scFv) were used as target cells, and Jurkat cells transfected with PD-1 and a luciferase gene under the control of NFAT elements were used as effector cells. Upon binding of the anti-CD3-scFv on the membrane of CHO cells to CD3 on the surface of Jurkat cells, activation signals were presented to Jurkat cells, thereby inducing the expression of luciferase. After PD-L1 on the surface of CHO cells was bound to PD-1 on the surface of Jurkat cells, inhibitory signals were delivered to Jurkat cells, and the expression of luciferase was inhibited. The binding of PD-1 to PD-L1 could be blocked by an anti-PD-1/anti-PD-L1 antibody, thereby abrogating the delivery of inhibitory signals, restoring the expression of luciferase, and generating fluorescent signals. Thus, the reporter-gene method was used to determine the biological activities of different types of anti-PD-1/anti-PD-L1 antibodies.

Firstly, CHO-PD-L1-CD3L cells was adjusted to a density of $5\times10^5$ cells/ml with a complete medium, added to a 96-well cell culture plate at 80 μl/well, and cultured in an incubator at 37°C and 5% $CO_2$ for 14-18 hours. The control sample and test samples were serially diluted with an assay medium. A Jurkat-PD1-NFAT cell suspension was adjusted to a cell density of $2\times10^6$ cells/ml with the assay medium. The culture plate was taken out, and the supernatant was aspirated and discarded. Then, serially diluted control sample and test samples at respective concentration gradients were added at 40 μl/well respectively, followed by the addition of the Jurkat-PD1-NFAT cell suspension at 40 μl/well. Incubation was performed in an incubator at 37°C and 5% $CO_2$ for 6 hours. At the end of the incubation, the 96-well culture plate was taken out, and 80 μl of BioLite staining solution was added to each well, and mixed uniformly by shaking under room temperature in the dark. The plate was read by a chemiluminescence microplate reader. With sample concentrations as the X-axis, and the average chemiluminescence signal values of the control sample and test samples as the Y-axis, a four-parameter fitting was performed to calculate the results.

With the sample prepared in Example 1 as control (the PD-1 biological activity of the control sample was set as 100%), the biological activities of the forced glucuronidation samples prepared in Example 3 were determined. Results of the assay of biological activities are shown in Table 3. The results of the analysis show that the biological activities of the respective samples were between 80-120% of the control sample.

Table 3. Results of analysis of biological activities of forced glucuronidation samples

| Sample type | PD-1 biological activity (%) |
| --- | --- |
| Control sample | 100 |
| Sample incubated at 37°C for 6 h | 107 |
| Sample incubated at 37°C for 12 h | 100 |
| Sample incubated at 37°C for 24 h | 105 |

The above results show that the binding activities and biological activities of the samples prepared in Example 3 were substantially consistent with the sample in Example 1.

Various modifications and equivalents may be made to the embodiments disclosed in the present application without departing from the spirit and scope of the disclosure. Unless otherwise specified in the context, any features, steps or embodiments of the embodiments of the present disclosure may be used in combination with any other features or embodiments.

**Claims**

1. A composition, wherein the composition comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab,

   wherein a portion of Pembrolizumab comprises a glucuronidation modification at the lysine at position 27 of a light chain having the amino acid sequence set forth in SEQ ID NO:2, and
   wherein the content of the light chain comprising the glucuronidation modification of Pembrolizumab in the composition is less than or equal to about 3%, expressed as a percentage of the content of the light chain comprising the glucuronidation modification of Pembrolizumab accounting for the total content of light chains of Pembrolizumab in the composition, which is calculated based on trypsin-cleaved peptide mass fingerprinting analysis.

2. The composition of claim 1, wherein the content of the light chain comprising the glucuronidation modification of Pembrolizumab in the composition is less than or equal to about 3.0%, less than or equal to about 2.9%, less than or equal to about 2.8%, less than or equal to about 2.7%, less than or equal to about 2.6%, less than or equal to about 2.5%, less than or equal to about 2.4%, less than or equal to about 2.3%, less than or equal to about 2.2%, less than or equal to about 2.1%, less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, or less than or equal to about 0.1%, expressed as a percentage of the content of the light chain comprising the glucuronidation modification of Pembrolizumab accounting for the total content of light chains of Pembrolizumab in the composition.

3. A pharmaceutical formulation comprising the composition of claim 1 or claim 2, and one or more pharmaceutically acceptable carriers.

4. A method for detecting Pembrolizumab comprising a glucuronidation modification in a Pembrolizumab-containing composition or pharmaceutical formulation, wherein the glucuronidation modification is located at the lysine at position 27 of a light chain having the amino acid sequence set forth in SEQ ID NO:2 of Pembrolizumab, the method comprising:

   subjecting the composition or pharmaceutical formulation to enzymatic cleavage with trypsin to obtain an enzymatically cleaved product; and

subjecting the enzymatically cleaved product to a peptide mass fingerprinting analysis, and determining the presence or absence of Pembrolizumab comprising the glucuronidation modification based on the first-order and second-order mass spectra;

wherein the composition or pharmaceutical formulation comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab.

5. The method of claim 4, wherein the peptide mass fingerprinting analysis is a peptide mass fingerprinting analysis using LC-MS/MS.

6. The method of claim 4 or 5, further comprising obtaining extracted ion chromatograms (XIC) of a peptide segment comprising the glucuronidation modification and an unmodified peptide segment after the peptide mass fingerprint analysis, and calculating the content of Pembrolizumab comprising the glucuronidation modification according to corresponding peak areas.

Figure 1

Figure 2

Relative abundance

0  10  20  30  40  50  60  70  80  90  100

Mass-to-charge ratio

y2+ 272. 17
b5++ 283. 14 *
y6++ 313. 18
y3+ 343. 21
y7++ 377. 22
b3+ 409. 17 *
b8++ 420. 69 *
b4+ 466. 20 *
y4+ 471. 27
y12+++ 499. 26
y9++ 505. 28
y5+ 528. 29
y13+++ 536. 95
b5+ 565. 26 *
y10++ 586. 81
y14+++ 591. 30
y15+++ 620. 31
y6+ 625. 34
b6+ 652. 29 *
y16+++ 674. 68
y11++ 679. 85
y17+++ 693. 69
y18+++ 722. 69
[ M ]++++ 730. 35
y12++ 748. 38
y7+ 753. 44
y19+++ 756. 38
y20+++ 785. 38
y13++ 804. 93
y22+++ 837. 42
y8+ 881. 50
y14++ 886. 46
y15++ 929. 97
y9+ 1009. 56
y16++ 1011. 51
b17++ 1019. 45 *
y17++ 1040. 02
y18++ 1083. 54
y19++ 1134. 05
y10+ 1172. 62
y20++ 1177. 57
y22++ 1255. 61
y11+ 1358. 69
y23++ 1380. 66 *

4+

A S K G Y S T S G Y S Y L H W Y Q Q K P G Q A P R

Figure 3

Europäisches
Patentamt
European
Patent Office
Office européen
des brevets

# EUROPEAN SEARCH REPORT

**Application Number**

EP 25 20 9266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/152356 A2 (REGENERON PHARMA [US]) 8 August 2019 (2019-08-08) * page 1; example A11 * | 4-6 | INV. C07K16/28 A61K39/395 |
| X | LIU Y. DIANA ET AL: "Challenges and Strategies for a Thorough Characterization of Antibody Acidic Charge Variants", BIOENGINEERING, vol. 9, no. 11, 3 November 2022 (2022-11-03), page 641, XP093378173, ISSN: 2306-5354, DOI: 10.3390/bioengineering9110641 * page 10 (end) * | 4-6 | |
| X | WO 2025/140495 A1 (QILU PHARMACEUTICAL CO LTD [CN]) 3 July 2025 (2025-07-03) * claims 1, 8 * | 1-3 | |

**TECHNICAL FIELDS SEARCHED (IPC)**

C07K

The present search report has been drawn up for all claims

| Place of search | Date of completion of the search | Examiner |
|---|---|---|
| The Hague | 10 April 2026 | Patti, Gabriele |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 20 9266

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2026

| Patent document cited in search report | Publication date | Patent family member(s) | Publication date |
|---|---|---|---|
| WO 2019152356 A2 | 08-08-2019 | AR 114569 A1 | 23-09-2020 |
| | | AU 2019213647 A1 | 23-07-2020 |
| | | BR 112020011797 A2 | 17-11-2020 |
| | | CA 3084181 A1 | 08-08-2019 |
| | | CN 112135839 A | 25-12-2020 |
| | | EA 202091577 A1 | 26-10-2020 |
| | | EP 3746472 A2 | 09-12-2020 |
| | | IL 276075 A | 31-08-2020 |
| | | JP 7301054 B2 | 30-06-2023 |
| | | JP 2021512280 A | 13-05-2021 |
| | | JP 2023134463 A | 27-09-2023 |
| | | KR 20200115467 A | 07-10-2020 |
| | | SG 11202005015X A | 29-06-2020 |
| | | TW 201940884 A | 16-10-2019 |
| | | TW 202348623 A | 16-12-2023 |
| | | US 2019234964 A1 | 01-08-2019 |
| | | US 2022276260 A1 | 01-09-2022 |
| | | WO 2019152356 A2 | 08-08-2019 |
| | | ZA 202003320 B | 25-10-2023 |
| WO 2025140495 A1 | 03-07-2025 | TW 202541843 A | 01-11-2025 |
| | | WO 2025140495 A1 | 03-07-2025 |

EPO FORM P0459

For more details about this annex : see Official Journal of the European Patent Office, No. 12/82

**REFERENCES CITED IN THE DESCRIPTION**

**Patent documents cited in the description**

- CN 202511191369X